# EUROPEAN PATENT SPECIFICATION

(11) **EP 0 296 503 B1**
(45) Date of publication and mention of the grant of the patent: **16.02.1994**
(21) Application number: 88109713.3
(22) Date of filing: 17.06.1988
(51) Int. Cl.: C07C 67/055, C07C 69/16

(54) **Process for producing glycol esters**
Verfahren zur Herstellung von Glykolestern
Procédé pour la fabrication d'esters de glycol

(30) Priority: 25.06.1987 JP 156496/87; 24.08.1987 JP 209897/87; 29.09.1987 JP 242571/87
(43) Date of publication of application: 28.12.1988
(73) Proprietor: IDEMITSU KOSAN COMPANY LIMITED, Tokyo 100 (JP)
(72) Inventor: Kezuka, Hiroaki c/o Idemitsu Kosan Co., Ltd., Kimitsu-gun, Chiba-ken, (JP); Kono, Takanori c/o Idemitsu Kosan Co., Ltd., Kimitsu-gun, Chiba-ken, (JP); Takizawa Toshiharu c/o Idemitsu Kosan Co. Ltd.,, Kimitsu-gun, Chiba-ken, (JP)
(74) Representative: Türk, Gille, Hrabal, Leifert

(56) References cited:
- PATENT ABSTRACTS OF JAPAN, vol. 10, no. 335 (C-384)(2391), 13 November 1986; & JP-A-61 140 545
- PATENT ABSTRACTS OF JAPAN, vol. 10, no. 13 (C-323)(2070), 18 January 1986; & JP-A-60 169 440
- PATENT ABSTRACTS OF JAPAN, vol. 8, no. 11 (C-205)(1448), 18 January 1984; & JP-A-58 177 936
- SOVIET INVENTIONS ILLUSTRATED, Section Chemical, Week D 37, abstract no. 67376, E 17, 21 October 1981, Derwent Publications Ltd., London, GB; & SU-A-789 506
- SOVIET INVENTIONS ILLUSTRATED, Section Chemical, Week E 05, abstract no. 09552, E17, 17 March 1982, Derwent Publications Ltd., London, GB; & SU-A-823 383
- CHEMICAL ABSTRACTS, vol. 85, no. 5, 1976, page 347, column 2, abstract no. 32441r, Columbus, Ohio, US; & JP-A-76 08210
- CHEMICAL ABSTRACTS, vol. 85, no. 19, 1976, page 489, column 1, abstract no. 142676d, Columbus, Ohio, US; & JP-A-76 82213

## Description

The present invention relates to a process for producing glycol esters to be used as chemical intermediate materials and solvents with high efficiency and in a high yield, by reacting lower aliphatic carboxylic acid and olefin in the presence of a specified catalyst and oxygen, which process is characterized in that a halogen ion source and/or a nitric acid ion source is added in the middle of the reaction. The present invention further relates to a process for producing glycol esters to be used as chemical intermediate materials, solvents and the like, with high efficiency by recovering the specified catalyst and recycling it, thereby reducing deterioration or deactivation of the catalyst.

### BACKGROUND OF THE INVENTION

A method of producing ethylene glycol monoester by introducing and reacting ethylene and oxygen at a temperature of less than 80°C in an organic acid solution containing a palladium salt, nitric acid or nitrous acid, and not less than an equimolar amount relative to the palladium salt of the organic acid salt of alkali or alkali earth metal is known (Japanese Patent Publication No. 14773/1970).

In addition, a method of producing glycol ester by introducing and reacting an internal olefin and oxygen in a carboxylic acid mixed solution containing palladium or a palladium salt, a nitrogen-containing oxide, and an alkali metal halide in the amount that the atomic ratio of halogen to palladium (halogen/palladium) is in the range of 0.5:1 to 5:1 has been proposed (Japanese Patent Kokai Koho Nos. 8210/1976 and 82213/1976).

Furthermore, a method of producing glycol ester by introducing and reacting an unsaturated compound and oxygen in lower aliphatic carboxylic acid containing a palladium catalyst and an oxygen-containing nitrogen compound, stopping the introduction of the unsaturated compound while at the same time introducing hydrogen to reduce the palladium catalyst, separating the reaction product, and recycling the residue obtained to the reaction is known (Japanese Patent Kokai Koho No. 177936/1983).

In addition, JP-A-61-140545 discloses a process for production of glycol esters by the reaction of a carboxylic acid with an olefin and oxygen in the presence of a catalyst comprising a Pd-component, an oxygen-containing nitrogen compound and a metal halide compound as main components. Furthermore, a method for preparing glycol acetates is disclosed in JP-A- 76-82213.

These methods, however, have a disadvantage in that on continuing the reaction, catalytic activity gradually drops, leading to a reduction in the selectivity of glycol esters, although the catalyst activity is high at an earlier stage of the reaction. Another disadvantage is that the catalyst is difficult to reproduce.

As a result of investigations to overcome the above problems, it has been found that in producing glycol esters by reacting lower aliphatic carboxylic acid, olefin and oxygen, part of the halogen added is converted into an organic halide during the reaction and the organic halide is removed from the reaction system and, therefore, the amount of halogen acting as a catalyst is decreased, and that the nitric acid ion is converted into gases such as NO, NO₂ in hydrogen reduction of the reaction solution and the gases are removed from the reaction system. Based on the findings, it has been discovered that if the lost halogen and nitric acid ion are replenished, even if the catalyst is used repeatedly, the activity of the catalyst does not drop and, therefore, glycol esters can be efficiently produced without reduction of the selectivity thereof.

### SUMMARY OF THE INVENTION

The present invention relates to a process for producing glycol esters by reacting lower aliphatic carboxylic acid and olefin in the presence of a catalyst containing (A) a palladium component, (B) a metal component selected from the metals of Periodic Table Groups IA, IB, IIA, IIB, VA, VIIB and VIII, (C) a halogen component and (D) a nitrogen-containing oxide, and oxygen, which process is characterized in that a halogen ion source and/or a nitric acid ion source is added in the course of the reaction. This is hereinafter referred to as "Process A".

The present invention further relates to a process as described above wherein the catalyst is added to the reaction system in the form of a catalyst solution which is obtained according to the following process steps:
(1) a step in which after the above disclosed reaction for producing the glycol esters, hydrogen is introduced to reduce the palladium catalyst;
(2) a step in which the reduced solution obtained is separated into reduced palladium and the reaction solution by filtration;
(3) a step in which the reaction solution is distilled to separate lower aliphatic carboxylic acid and glycol esters; and
(4) a step in which to the reduced palladium obtained in the step (2) above, lower aliphatic carboxylic acid, the distillation residue obtained in the step (3), a halogen ion source and/or a nitric acid ion source are added to prepare a catalyst solution, and the catalyst solution is introduced in the reaction system. This is hereinafter referred to as "Process B".

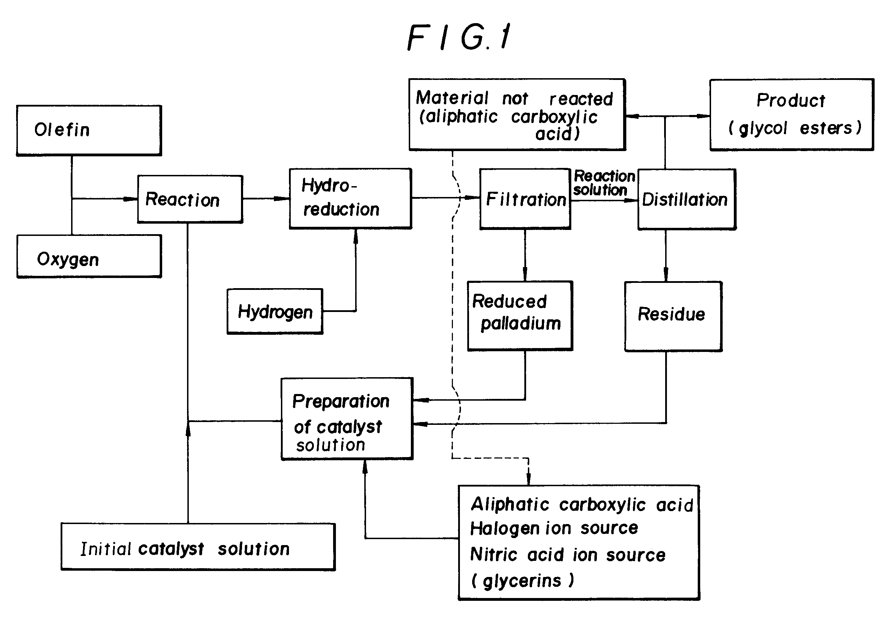

### BRIEF DESCRIPTION OF THE DRAWING

The Figure is a flow diagram of an embodiment of the present invention.

### DETAILED DESCRIPTION OF THE INVENTION

In accordance with the process of the present invention, carboxylic acid, olefin and oxygen are reacted in the presence of the aforementioned catalyst to produce the desired glycol esters. Various carboxylic acids and olefins can be used as the starting materials depending on the desired glycol esters. For example, as the carboxylic acid, aliphatic carboxylic acids represented by the general formula:

RCOOH (I)

(wherein R is a lower alkyl group). Specific examples are acetic acid, propionic acid, butyric acid and isobutyric acid.

As the olefin, carbon-carbon double bond-containing unsaturated compounds represented by the general formula:

(R¹)(R²)C=C(R³)(R⁴) (II)

(wherein R¹ to R⁴ are each hydrogen, a lower alkyl group, an aryl group, or an alkenyl group) can be used. In particular, those in which R¹ is hydrogen or a lower alkyl group, and R², R³ and R⁴ are each hydrogen are preferred. Specific examples are α-olefin such as ethylene, propylene and butene-1.

The oxygen is not limited to pure oxygen, but includes air. In addition, mixed gases of oxygen and nitrogen, carbon dioxide or saturated hydrocarbons such as methane, ethane and propane can be used.

The amounts of the starting materials are not critical and can be determined appropriately taking into consideration the type of the desired glycol ester. The molar ratio of olefin to oxygen (olefin/oxygen) charged (charge ratio) is usually 1.0:1 to 3.0:1, preferably 1.6:1 to 2.2:1 and more preferably 1.8:1 to 2.0:1. If the charge molar ratio is in excess of 3.0:1, degradation of the catalyst is accelerated. On the other hand, if it is less than 1.0:1, they are in the range of explosion.

The catalyst to be used in the present invention comprises, as described above, the components (A), (B), (C) and (D). The palladium component as the component (A), is used as metallic palladium or an inorganic compounds such as sodium chloropalladium, palladium nitrate, palladium acetate, palladium chloride and palladium bromide. In particular, metallic palladium is preferred. Although it is preferred for the palladium component to be used as such, it can be used in the state that it is deposited on a support such as active carbon. Although the amount of the palladium component compounded is not critical, it is usually desirable to add in an amount of 0.001 to 10% by weight based on the weight of the solvent.

The Periodic Table Group IA, IB, IIA, IIB, VA, VIIB or VIII metal component as the component (B) is used in the form of metal or an inorganic compound. Preferred metals are alkali metals, alkali earth metals, copper, manganess, zinc, cobalt and bismuth. Particularly preferred metals are alkali metals and alkali earth metals. More particularly preferred is lithium.

The amount of the component (B) compounded is usually 0.1 to 15 mol, preferably 0.2 to 15 mol and particularly preferably 1 to 10 mols per gram atom of the above palladium.

The halogen component (C) is used as an inorganic compound, and as the halogen, chlorine is preferred. The halogen component can be used in combination with the above components (A) and (B). For example, as the component (C), the halides of palladium, alkali metals or alkali earth metals can be used. The amount of the halogen component compounded is usually 0.1 to 15 mol, preferably 0.2 to 5 mol and particularly preferably 2.0 to 4.0 mol per gram atom of the palladium. If the amount of the halogen component is less than 0.1 mol per gram atom of the palladium, the solubility of palladium drops. On the other hand, if it is in excess of 15 mol, the conversion of olefin such as propylen and the like is decreased.

Specific examples of the nitrogen-containing oxide (D) include nitrogen oxides such as NO, NO₂ and N₂O₃, nitric acid, fuming nitric acid, the nitric acid salts and nitrous acid salts of alkali metals and alkali earth metals, nitrous acid esters such as alkyl nitrite. Of these compounds, nitric acid, nitric acid salts such as lithium nitrate and sodium nitrate, and nitrous acid esters such as ethyl nitrite and propyl nitrite are preferred.

The amount of the nitrogen-containing compound compounded is usually 0.1 to 15 mol, preferably 0.2 to 5 mol and particularly preferably 0.5 to 3 mol per gram atom of palladium. If the amount of the nitrogen-containing compound compounded is less than 0.1 mol, the solubility of palladium drops. On the other hand, if it is in excess of 15 mol, deactivation of palladium is undesirably increased.

In the present invention, the catalyst comprising the above components (A) to (D) is used in the state that it is dissolved in lower aliphatic carboxylic acid. In this case, if glycerines are used in combination, the catalyst in the reaction system is increased in uniformity and maintained in the active state. In addition, when the desired glycol esters are continuously produced by recovering the catalyst component, reproducing it and recycling it, a distillation residue containing the catalyst component can be effectively recovered and the glycol esters can be produced in a high yield.

The glycerins as used herein mean glycerine, glycerine esters and the like as represented by the following general formula: 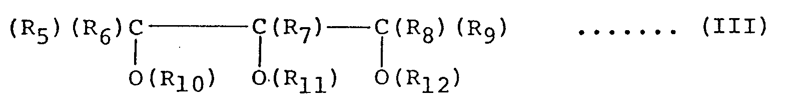
(wherein R₅ to R₉ are each a hydrogen atom or an alkyl group, and R₁₀ to R₁₂ are each a hydrogen atom or an acyl group represented by R'CO- (wherein R' is an alkyl group).

Glycerine esters include acetins such as glycerine monoacetate, glycerine diacetate, glycerine triacetate (triacetin), and butyrins such as glycerine monobutyrate, glycerine dibutyrate and glycerine tributyrate. The amount of the glycerines used is not critical, and it is sufficient for the glycerines to be used in an effective amount to hold the catalyst uniformly in the reaction system.

Production of glycol esters by reacting carboxylic acid, olefin and oxygen by the use of the catalyst solution as described above can be carried out under various conditions. The reaction temperature is usually 20 to 120°C and preferably 20 to 80°C. If the reaction temperature is in excess of 120°C, the selectivity of the glycol esters is undesirably decreased. The reaction pressure is usually from atmospheric pressure to 9,8·10⁵ Pa (10 kg/cm²G) and preferably atmospheric pressure to 4,9·10⁵ Pa (5 kg/cm²G). If the reaction pressure is in excess of 9,8·10⁵ Pa (10 kg/cm²G), the selectivity is undesirably decreased.

The reaction can be carried out by any of the batch method, the semi-continuous method and the continuous method. The state of the reaction system may be either a liquid phase or a mixed state of a gas phase and a liquid phase. The state of the catalyst in the reaction system may be either homogeneous or heterogeneous, but it is desirably homogeneous.

To the above reaction system, a diluent can be added. For example, an inert solvent such as alcohols and esters saturated hydrocarbon gas, cyclic hydrocarbon gas, or inert gas such as nitrogen gas can be used.

In Process A of the present invention, a halogen ion source and/or a nitric acid ion source is added in the course of the reaction.

Examples of the halogen ion source include hydrogen chloride. The halogen ion source is added in an amount corresponding to the deficiency of the catalyst concentration. Examples of the nitric acid ion source include, as well as fuming nitric acid and nitric acid, nitrogen oxide such as NO, NO₂ and N₂O₃. The nitric acid ion source is added in an amount corresponding to the deficiency of the catalyst concentration.

In connection with a method of addition, the above compounds are added, directly or after dissolving in lower aliphatic carboxylic acid, and continuously or in several steps. In the case of the continuous production method, when a plurality of reactors are connected in series, the halogen ion source and/or the nitric acid ion source is continuously or succesively added to each reactor. In the case of a tubular reactor, one or a plurality of injection openings are provided at a suitable location between the inlet and outlet of the reactor, and the halogen ion source and/or the nitric acid ion source is introduced continuously or succesively.

If the halogen ion source and/or the nitric acid ion source is added excessively from the beginning, the amounts of organic halogen compounds and/or organic nitrogen-containing compounds formed are increased, leading to a reduction in the selectivity, and further the reduction of the olefin conversion and deactivation of the palladium are undesirably caused.

In Process B of the present invention, as described above, the steps (1) to (4) are carried out in the order, whereby the catalyst is recovered and recycled, and the desired glycol esters are produced efficiently.

Glycol esters obtained by the process of the present invention are basically represented by the general formula: 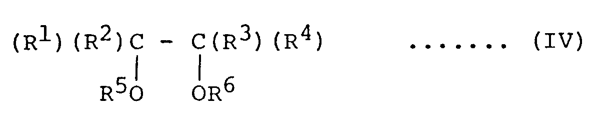
(wherein R¹, R², R³ and R⁴ are the same as defined in the general formula (II), and R⁵ and R⁶ are each hydrogen or an acyl group (RCO- wherein R is the same as R of the general formula of the aforementioned aliphatic carboxylic acid), and the number of hydrogen is 0 or 1). In addition, those derived from substitution of the above glycol esters by a group or groups are included. As by-products in the reaction, aldehyde and ketone are formed.

The present invention will hereinafter be explained with reference to a continuous process for production of glycol esters which is a suitable embodiment of the present invention.

The Figure is a flow diagram of the process. As apparent from the flow diagram, olefin and oxygen are introduced in a catalyst solution containing a carboxylic acid starting material, and then reacted. Thereafter, (1) a step in which hydrogen is introduced to reduce the palladium catalyst, and (2) a step in which the reduced solution thus obtained is separated into reduced palladium and the reaction mixture, and (3) a step in which the reaction mixture is distilled to separate aliphatic carboxylic acid and glycol esters are carried out. Then, (4) a step in which the reduced palladium obtained in the step (2), aliphatic carboxylic acid, the distillation residue obtained in the step (3), a halogen ion source and a nitric acid ion source are mixed to prepare a catalyst solution, and this catalyst solution is introduced in the reaction system is carried out. As carboxylic acid to be used as a solvent in the preparation of the catalyst solution at the step (4), carboxylic acid as separated at the step (3) may be used, or a fresh carboxylic acid starting material may be supplied. The catalyst solution thus prepared is introduced in the above reaction system.

Separation and recovery of the catalyst used in the process of the present invention can be carried out as follows:
In the first place, after the reaction, hydrogen is introduced in the reaction system to reduce the palladium catalyst to metallic palladium, and the metallic palladium is recovered by a separation technique such as filtration. The hydrogen to be introduced is not limited to pure hydrogen gas, and mixed gases resulting from dilution of hydrogen with nitrogen, helium, methane, ethane, propane can also be used. Hydrogen introduction conditions are not critical as long as a degraded palladium catalyst can be sufficiently reduced. In general, hydrogen is introduced under atmospheric pressure or under pressure, at a temperature of 20 to 150°C, preferably 20 to 80°C for about 5 to 120 minutes.

The reaction mixture from which the reduced palladium has been separated by filtration is distilled to obtain the reaction product and unreacted starting materials, and at the same time, a distillation residue is recovered. The distillation residue contains carboxylic acid salts represented by the general formula: 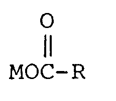
(wherein M is a Group IA, IB, IIA, VA, VIIB or VIII metal and R is an alkyl group) and metal halides represented by the general formula:

MX

(wherein M is a Group IA, IB, IIA, VA, VIIB or VIII metal and X is a halogen), and thus can be reused as one of the catalyst component.

The nitrogen-containing oxide added to the catalyst solution is consumed in the reaction and also in hydrogen reduction. Thus, when a metal salt of nitric acid is used, it is recovered as a distillation residue along with the metal halide. These may be recovered by extraction or crystallization in place of distillation. When a nitrous acid ester is used, the alkyl radical is recovered as an alcohol by distillation. It may be recovered by extraction in place of distillation.

To the reduced palladium and distillation residue thus recovered are added a halogen ion source and a nitric acid ion source, and low aliphatic carboxylic acid as a solvent and if necessary, glycerines to prepare a catalyst solution. That is, the halogen ion source, the nitric acid ion source and the solvent are added to the distillation residue and uniformly dissolved therein, and then the reduced palladium is added to the resulting solution and stirred to prepare the catalyst solution.

As the halogen ion source to be added to the recovered catalyst, hydrogen chloride and hydrochloric acid can be used. The halogen ion source is added in an amount corresponding to the deficiency of the catalyst concentration. As the nitric acid ion source, as well as fuming nitric acid and nitric acid, nitrogen oxides such as NO, NO₂ and N₂O₃ can be used.

The reason why nitric acid ion is insufficient is that at the time of hydrogen reduction of the reaction solution, it is converted into gases such as NO and NO₂, and removed from the reaction system like the halogen.

In accordance with Process A of the present invention, a halogen ion source and/or a nitric acid ion source is added in the course of the reaction in production of glycol esters, whereby the selectivity is increased, the concentration of the product in the reaction mixture is increased and thus the separation of the product from the reaction solvent is facilitated.

In Process B of the present invention, the reaction can be carried out continuously, and the catalyst can be recycled continuously reducing the degradation or deactivation thereof. If glycerines are added to the reaction system, the catalyst can be recovered with high efficiency and the uniformity of the catalyst in the reaction system is increased and, therefore, the reaction can be carried out in an active state. Accordingly, glycol esters such as ethylene glycol ester and propylene glycol ester, which are useful as chemicals and solvents, can be obtained in a high yield. Moreover, this method is very useful for practical use because it is very simplified in operation and the loss of palladium of the catalyst is small.

The present invention is described in greater detail with reference to the following examples.

### EXAMPLE 1

(1) 0.214 g of metallic palladium, 0.250 g of lithium chloride, 0.281 g of lithium nitrate and 120 ml of acetic acid were placed in a 200-milliliter four-necked flask equipped with a stirring blade, a cooler, a thermometer and a gas introduction tube, and stirred while heating on an oil bath maintained at 60°C to prepare a catalyst solution.
(2) Propylene and oxygen were introduced with stirring in the catalyst solution prepared above through a gas introduction tube at rates of 24.5 ml/min and 13 ml/min, respectively, and reacted for 4 hours.
(3) In the course of the reaction, i.e., at a point of 2 hours from the start of the reaction, 1.55 g of an acetic acid solution (2.9%) of hydrogen chloride was added. After the reaction, the product was analyzed and quantitatively measured by gas chromatography. The results are shown in Table 1.

**Table 1**

| Product | Amount (m mol) | Selectivity (%) |
|---|---|---|
| PGMA*¹ | 216.3 | 87.5 |
| PGDA*² | 5.3 | 2.1 |
| Acetone | 17.5 | 7.1 |
| Propylidene diacetate | 7.6 | 3.1 |

| | | |
|---|---|---|
| *1 PGMA: Propylene glycol monoacetate | | |
| *2 PGDA: Propylene glycol diacetate | | |

### COMPARATIVE EXAMPLE 1

The procedure of Example 1 was repeated with the exception that in the course of the reaction, the acetic acid solution of hydrogen chloride was not added. The results are shown in Table 2.

**Table 2**

| Product | Amount (m mol) | Selectivity (%) |
|---|---|---|
| PGMA | 205.0 | 79.3 |
| PGDA | 8.3 | 3.2 |
| Acetone | 28.3 | 10.9 |
| Propylidene diacetate | 11.6 | 4.5 |

### EXAMPLE 2

The procedure of Example 1 was repeated with the exception that in the course of the reaction, a solution of 0.26 g of fuming nitric acid in 15 ml of acetic acid was used in place of the acetic acid solution (2.9%) of hydrogen chloride. The results are shown in Table 3.

**Table 3**

| Product | Amount (m mol) | Selectivity (%) |
|---|---|---|
| PGMA | 204.1 | 87.5 |
| PGDA | 6.1 | 2.6 |
| Acetone | 13.1 | 5.6 |
| Propylidene diacetate | 6.8 | 2.9 |

### EXAMPLE 3

(1) The procedure of (1) of Example 1 was repeated with the exception that the amount of lithium chloride used was changed from 0.250 g to 0.187 g, and the amount of lithium nitrate used was changed from 0.281 g to 0.148 g.
(2) Propylene and oxygen were introduced with stirring in the catalyst solution prepared above through the gas introduction tube at rates of 24 ml/min and 12 ml/min, respectively, and reacted for 2 hours.
(3) Thereafter, the introduction of propylene and oxygen was stopped, and nitrogen was introduced at a rate of 30 ml/min for 5 minutes. Then, the introduction of nitrogen was stopped, and hydrogen was introduced at a rate of 25 ml/min for 1 hour to reduce the palladium component into metallic palladium. After the introduction of hydrogen was stopped, nitrogen was again introduced at a rate of 30 ml/min for 5 minutes. About 2 ml of the supernatant was sampled and the product was quantitatively measured by gas chromatography.
(4) After the reduction, the reduced palladium was left in the flask, and the reaction mixture was withdrawn while filtering through a glass filter.
(5) The reaction mixture withdrawn in (4) above was distilled under reduced pressure to separate acetic acid and the product.
(6) To the distillation residue obtained in (5) above, 0.15 g of hydrochloric acid (36%), 0.16 g of fuming nitric acid and 120 ml of acetic acid were added, and the resulting mixture is introduced in the flask in which the reduced palladium was left and stirred while heating on an oil bath maintained at 60°C to prepare a catalyst solution. About 2 ml of the catalyst solution was sampled and the residual amount of the product was quantitatively measured by gas chromatography.

Thereafter, the steps (2) to (6) were repeated. The amount and selectivity after the reaction was conducted four times are shown in Table 4.

**Table 4**

| | Number of Reactions | | | |
|---|---|---|---|---|
| | 1 | 2 | 3 | 4 |
| Amount of PGMA (m mol) | 102.7 | 99.5 | 97.5 | 96.5 |
| Amount of PGDA (m mol) | 3.3 | 3.3 | 3.6 | 3.4 |
| Selectivity of (PGMA + PGDA) (%) | 85.8 | 86.7 | 84.3 | 83.1 |
| Residual Amount of PGMA (m mol) | 29.7 | 52.2 | 22.7 | - |
| Residual Amount of PGDA (m mol) | 0.7 | 1.6 | 0.8 | - |

### COMPARATIVE EXAMPLE 2

The reaction was repeated in the same manner as in Example 3 except that at the step (6), hydrochloric acid (36%) was not used. The amount and selectivity after the reaction was repeated 3 times are shown in Table 5.

**Table 5**

| | Number of Reaction | | |
|---|---|---|---|
| | 1 | 2 | 3 |
| Amount of PGMA (m mol) | 96.6 | 94.7 | 89.8 |
| Amount of PGDA (m mol) | 2.4 | 2.9 | 5.2 |
| Selectivity of (PGMA + PGDA) (%) | 86.6 | 82.1 | 81.4 |
| Residual Amount of PGMA (m mol) | 33.8 | 52.5 | - |
| Residual Amount of PGDA (m mol) | 0.6 | 2.3 | - |

### EXAMPLE 4

The procedure of Example 3 was repeated with the exception that at the step(1), the amount of lithium chloride was changed from 0.187g to 0.255g and the amount of lithium nitrate was changed from 0.148g to 0.278g, at the step(6), 1.50g of hydrogen chloride in acetic acid (2.9%) was used in place of hydrochloric acid and the amount of fuming nitric acid was changed from 0.16g to 0.25g. The amount and selectivity after the reaction was repeated 5 times are shown in Table 6.

**Table 6**

| | Number of Reactions | | | | |
|---|---|---|---|---|---|
| | 1 | 2 | 3 | 4 | 5 |
| Amount of PGMA (m mol) | 112.8 | 108.3 | 104.3 | 102.5 | 100.3 |
| Amount of PGDA (m mol) | 2.7 | 3.2 | 4.2 | 2.9 | 3.2 |
| Selectivity of (PGMA + PGDA) (%) | 93.4 | 93.1 | 93.6 | 93.3 | 93.0 |
| Residual Amount of PGMA (m mol) | 17.4 | 26.7 | 18.6 | 20.1 | |
| Residual Amount of PGDA (m mol) | 0.3 | 0.6 | 0.6 | 0.4 | |

### EXAMPLE 5

(1) 0.214 g of metallic palladium, 0.256 g of lithium chloride, 0.275 g of lithium nitrate, 110 ml of acetic acid and 10 ml of triacetylene were placed in a 200-milliliter four-necked flask equipped with a stirring blade, a cooler, a thermometer and a gas introduction tube, and stirred while heating on an oil bath maintained at 60°C to prepare a catalyst solution.
(2) Propylene and oxygen were introduced in the catalyst solution prepared above through the gas introduction tube while stirring at rates of 24 ml/min and 12 ml/min, respectively, and reacted for 2 hours.
(3) Thereafter, the introduction of propylene and oxygen was stopped, and nitrogen was introduced at a rate of 30 ml/min for 5 minutes. Then the introduction of nitrogen was stopped, and hydrogen was introduced at a rate of 25 ml/min for 1 hour to convert the palladium component into metallic palladium. After the introduction of hydrogen was stopped, nitrogen was again introduced at a rate of 30 ml/min for 5 minutes. About 2 ml of the supernatant was sampled and the product was quantitatively measured by gas chromatography. 119.5 m mol of PGMA and 2.3 m mol of PGDA (selectivity of (PGMA + PGDA), 93.7%) were formed.
(4) After the reduction, the reduced palladium was left in the flask, and the reaction mixture was withdrawn while filtering with a glass filter.
(5) The reaction mixture withdrawn in (4) above was distilled under reduced pressure to separate acetic acid and the product.
(6) To the distillation residue of (5) above, 1.49 g of an acetic acid solution (2.9%) of hydrogen chloride, 0.29 g of fuming nitric acid and 110 ml of acetic acid were added, and they were then introduced in the flask in which the reduced palladium was left and stirred while heating on an oil bath maintained at 60°C to prepare a catalyst solution. About 2 ml of the catalyst solution was sampled and the residual amount of the product was quantitatively measured. In the residue, 5.8 m mol of PGMA and 0.2 m mol of PGDA remained.
(7) The solution prepared in (6) above was again used in the reaction, and the reaction was repeated totally four times. About 2 ml of the solution was sampled and quantitatively measured by gas chromatography. 113.1 m mol of PGMA and 2.6 m mol of PGDA (selectivity of (PGMA + PGDA), 93.4%) were formed.

### EXAMPLE 6

The reaction was repeated in the same manner as in Example 5 except that the amount of acetic acid used in the step (1) was changed from 110 ml to 100 ml, the amount of triacetin used was changed from 10 ml to 20 ml, and the amount of acetic acid used in the step (6) was changed from 110 ml to 100 ml. The amount and selectivity, and residual amount of the product in the distillation residue at the first and fifth reactions are shown in Table 7.

**Table 7**

| | Number of Reactions | |
|---|---|---|
| | 1 | 5 |
| Amount of PGMA (m mol) | 116.2 | 110.8 |
| Amount of PGDA (m mol) | 1.8 | 1.7 |
| Selectivity of (PGMA + PGDA) (%) | 93.9 | 93.8 |
| Residual Amount of PGMA (m mol) | 4.7 | |
| Residual Amount of PGDA (m mol) | 0.2 | |

### EXAMPLE 7

0.216 g of metallic palladium, 0.265 g of lithium chloride, 0.440 g of lithium nitrate, 120 ml of acetic acid and 5 ml of diacetylene were placed in the same apparatus as used in Example 5, and a catalyst solution was prepared in the same manner as in Example 5. Using the catalyst solution, the reaction and the reduction were carried out in the same manner as in Example 5. 110.5 m mol of PGMA and 0.9 m mol of PGDA (selectivity of (PGMA + PGDA), 96.5%) were formed.

Distillation was carried out in the same manner as in the steps (4) and (5) of Example 5. The residue contained 5.8 m mol of PGMA and 0.2 m mol of PGDA.

To the distillation residue, 1.49 g of an acetic acid solution (2.9%) of hydrogen chloride, 0.39 g of fuming nitric acid and 120 ml of acetic acid were added to prepare a catalyst solution in the same manner as in the steps (6) and (7) of Example 5. Using the catalyst solution, the reaction was repeated totally 4 times.

105.8 m mol of PGMA and 1.5 m mol of PGDA (selectivity of (PGMA + PGDA), 96.0%) were formed.

## Claims

1. A process for producing glycol esters by reacting a lower aliphatic carboxylic acid and an olefin in the presence of a catalyst and oxygen, said catalyst containing (A) a palladium component, (B) a metal component selected from the metals of Groups IA, IB, IIA, IIB, VA, VIIB and VIII of the Periodic Table, (C) a halogen component and (D) a nitrogen-containing oxide, which process is characterized in that a halogen ion source and/or a nitric acid ion source is added in the course of the reaction.

2. The process as claimed in Claim 1 wherein the catalyst is added to the reaction system in a catalyst solution which is obtained according to the following process steps:
(1) a step in which after the reaction of Claim 1, hydrogen is introduced to reduce the palladium catalyst;
(2) a step in which the reduced solution thus obtained is separated into reduced palladium and the reaction solution by filtration;
(3) a step in which the reaction solution is distilled to separate the lower aliphatic carboxylic acid and the glycol ester; and
(4) a step in which a lower aliphatic carboxylic acid, the distillation residue obtained in the step (3), a halogen ion source and/or a nitric acid ion source are added to the reduced palladium obtained in the step (2) to prepare a catalyst solution, and the catalyst solution is added to the reaction system.

3. The process as claimed in Claim 1 wherein the lower aliphatic carboxylic acid and the olefin are reacted in the presence of glycerine compound.

## Patentansprüche

1. Verfahren zur Herstellung von Glycolestern durch Umsetzen einer niederen aliphatischen Carbonsäure und eines Olefins in Gegenwart eines Katalysators und Sauerstoff, wobei der Katalysator enthält (A) eine Palladiumkomponente, (B) eine Metallkomponente ausgewählt aus den Metallen der Gruppen IA, IB, IIA, IIB, VA, VIIB und VIII des Periodensystems, (C) eine Halogenkomponente und (D) ein stickstoffhaltiges Oxid, wobei das Verfahren dadurch gekennzeichnet ist, daß eine Halogenionenquelle und/oder eine Salpetersaureionenquelle im Verlauf der Reaktion zugegeben wird.

2. Verfahren nach Anspruch 1, worin der Katalysator dem Reaktionssystem in einer Katalysatorlösung zugegeben wird, die gemäß den folgenden Verfahrensstufen erhalten wird:
(1) eine Stufe, in der, nach der Reaktion von Anspruch 1, Wasserstoff eingeleitet wird, um den Palladiumkatalysator zu reduzieren,
(2) eine Stufe, in der die so erhaltene reduzierte Lösung durch Filtration in reduziertes Palladium und die Reaktionslösung aufgetrennt wird,
(3) eine Stufe, in der die Reaktionslösung destilliert wird, um die niedere aliphatische Carbonsäure und den Glycolester zu trennen, und
(4) eine Stufe, in der eine niedere aliphatische Carbonsäure, der in der Stufe (3) erhaltene Destillationsrückstand, eine Halogenionenquelle und/oder eine Salpetersäureionenquelle zu dem in der Stufe (2) erhaltenen reduzierten Palladium zugegeben werden, um eine Katalysatorlösung herzustellen, und die Katalysatorlösung zu dem Reaktionssystem zugegeben wird.

3. Verfahren nach Anspruch 1, worin die niedere aliphatische Carbonsäure und das Olefin in Gegenwart einer Glycerinverbindung umgesetzt werden.

## Revendications

1. Procédé de production d'esters de glycol, qui consiste à faire réagir un acide carboxylique aliphatique inférieur et une oléfine, en présence d'un catalyseur et d'oxygène, ledit catalyseur contenant (A) un composé du palladium, (B) un **dérivé** métallique choisi parmi les métaux des groupes IA, IB, IIA, IIB, VA, VIIB et VIII de la classification périodique, (C) un **dérivé** d'halogène et (D) un oxyde azoté, ce procédé étant caractérisé en ce que l'on ajoute, au cours de la réaction, une source d'ions halogènes et/ou une source d'ions d'acide nitrique.

2. Procédé selon la revendication 1, dans lequel le catalyseur est ajouté au système réactionnel sous forme d'une solution de catalyseur que l'on obtient selon les étapes opératoires suivantes :
(1) une étape dans laquelle, après la réaction de la revendication 1, on introduit de l'hydrogène pour réduire le catalyseur au palladium;
(2) une étape dans laquelle la solution réduite ainsi obtenue est séparée en palladium réduit et en solution réactionnelle par filtration;
(3) une étape dans laquelle la solution réactionnelle est distillée pour séparer l'acide carboxylique aliphatique inférieur et l'ester de glycol; et
(4) une étape dans laquelle un acide carboxylique aliphatique inférieur, le résidu de distillation obtenu dans l'étape (3), une source d'ions halogènes et/ou une source d'ions d'acide nitrique sont ajoutés au palladium réduit obtenu dans l'étape (2) pour préparer une solution de catalyseur, et la solution de catalyseur est ajoutée au système réactionnel.

3. Procédé selon la revendication 1, dans lequel la réaction entre l'acide carboxylique aliphatique inférieur et l'oléfine se fait en présence d'un composé glycérine.
